# Europäisches Patentamt
# European Patent Office
# Office européen des brevets

(19)

(11) Veröffentlichungsnummer: **0 038 999**
**B2**

(12) **NEUE EUROPÄISCHE PATENTSCHRIFT**

(45) Veröffentlichungstag der neuen Patentschrift:
**18.12.85**

(51) Int. Cl.⁴: **C 07 C 143/40**

(21) Anmeldenummer: **81102897.6**

(22) Anmeldetag: **15.04.81**

(54) Verfahren zur Herstellung von 2-Chlor-5-formylbenzolsulfonsäure.

(30) Priorität: **26.04.80 DE 3016186**

(43) Veröffentlichungstag der Anmeldung:
**04.11.81 Patentblatt 81/44**

(45) Bekanntmachung des Hinweises auf die Patenterteilung:
**13.07.83 Patentblatt 83/28**

(45) Bekanntmachung des Hinweises auf die Entscheidung
über den Einspruch:
**18.12.85 Patentblatt 85/51**

(84) Benannte Vertragsstaaten:
**CH DE FR GB LI**

(56) Entgegenhaltungen:
**DE - A - 1 923 267**
**DE - A - 2 201 857**
**DE - A - 2 262 531**
**DE - C - 98 229**

**Fieser & Fieser: Organische Chemie, Weinheim 1968, S. 25-26**
**Fierz-David & Blangey: Grundlegende Operationen der Farbenchemie, Wien 1947, S. 32**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Marzolph, Gerhard, Dr., Roggendorfstrasse 65, D-5000 Köln 80 (DE)**
Erfinder: **Blank, Heinz Ulrich, Dr., Am Geusfelde 35, D-5068 Odenthal (DE)**

EP 0 038 999 B2

## Beschreibung

Die Erfindung betrifft ein Verfahren zur Herstellung von 2-Chlor-5-formylbenzolsulfonsäure durch Umsetzung von 4-Chlorbenzaldehyd mit Oleum bei erhöhter Temperatur.

Aus der DE-OS 1 923 267 und der DE-OS 2 525 684 ist es bekannt, Methoxy-substituierte Benzaldehyde zu sulfonieren. Hierbei können sehr milde Sulfonierungsbedingungen angewandt werden, die zeigen, dass zwischen der ortho- und der para-Verbindung kaum Unterschiede in der Reaktivität bestehen. So wird in DE-OS 1 923 267 bereits mit 5%igem Oleum und einem unterstöchiometrischen Verhältnis von 0,42 Mol $SO_3$ pro Mol p-Methoxy-benzaldehyd bei 90°C in einer 7stündigen Reaktion sulfoniert. Das unterstöchiometrische Verhältnis zeigt hierbei, dass ausser dem $SO_3$ bereits die als Lösungsmittel dienende Schwefelsäure sulfonierend wirkt. In der DE-OS 2 525 684 wird 2,3-Dimethoxy-benzaldehyd mit einer äquimolaren Menge $SO_3$ in Form von zunächst von 5%igem und dann nachgesetztem 25%igem Oleum bei 20°C sulfoniert.

Im Gegensatz dazu ist ein Chlor-substituierter Benzaldehyd generell sehr viel schwerer zu sulfonieren und zeigt darüberhinaus deutliche Reaktivitätsunterschiede zwischen der ortho- und der para-Verbindung.

So beschreibt die DE-PS 98 229 die Sulfonierung eines Gemisches aus 0,61 Mol-% ortho-Chlor- und 0,39 Mol-% para-Chlor-benzaldehyd. Hierbei wird bei 85°C und 30%igem Oleum in 45 Minuten und einem stöchiometrischen Verhältnis von 4,3 Mol $SO_3$ zu 1 Mol des Gemisches nur der ortho-Chlor-benzaldehyd umgesetzt. Zum Vergleich sei hierzu die DE-PS 91 315 herangezogen, in der reiner ortho-Chlor-benzaldehyd bei maximal 120°C mit 25%igem Oleum bei einem stöchiometrischen Verhältnis von 1,1 Mol $SO_3$ pro Mol ortho-Chlor-benzaldehyd sulfoniert wird. Hieraus ergibt sich, dass der ortho-Chlor-benzaldehyd leicht sulfoniert wird und weiterhin, dass unter Zugrundelegung des 1,1 molaren $SO_3$-Überschusses laut DE-PS 91 315 nach völligem Umsatz des ortho-Chlor-benzaldehyds in DE-PS 98 229 dort für eine eventuelle Sulfonierung des verbleibenden para-Chlor-benzaldehyds ein $SO_3$-Überschuss von 5,17 Mol pro Mol para-Chlor-benzaldehyd errechnet werden kann. Dennoch findet laut DE-PS 98 229 unter diesen Umständen eine Sulfonierung des para-Chlor-benzaldehyds nicht statt.

Die Erkenntnis der schweren Sulfonierbarkeit des para-Chlor-benzaldehyds wird auch deutlich in DE-OS 2 201 857, wo mit vergleichbar hohem Überschuss von 4,5 Mol $SO_3$ pro Mol para-Chlor-benzaldehyd gearbeitet wird und zur Erzwingung der Reaktion auf 65%iges Oleum übergegangen wird. Zur Vermeidung eines oxidativen Angriffs arbeitet diese DE-OS bei der niedrigen Temperatur von 60°C und nimmt hierfür die wirtschaftlich ungünstige lange Reaktionszeit von 20 Stunden und die nur mässige Ausbeute von 75% in Kauf. Dass solche oxidativen Angriffe zu befürchten waren, geht aus Gilbert, Sulfonation and Related Reactions, Interscience 1965, Seite 31/32 hervor, worin die Oxidation von Aliphaten und Cycloaliphaten mit Sulfonierungsagenzien wie Oleum beschrieben ist und weiter aus DE-PS 65 182, 65 375 und 65 453, worin die oxidative Hydroxylierung von Oxogruppen tragenden Aromaten, wie Anthrachinon, mit $SO_3$ und hochkonzentriertem Oleum beschrieben sind.

Es wurde nun ein Verfahren zur Herstellung von 2-Chlor-5-formylbenzolsulfonsäure durch Umsetzung von 4-Chlorbenzaldehyd mit einem Überschuss an Oleum bei erhöhter Temperatur gefunden, das dadurch gekennzeichnet ist, dass man die Umsetzung bei Temperaturen von 70 bis 110°C und bei einer Oleumkonzentration von 35–70 Gew.% durchführt.

Nach dem erfindungsgemässen Verfahren wird die Umsetzung bevorzugt bei Temperaturen von 75 bis 90°C, besonders bevorzugt bei 80 bis 85°C, durchgeführt.

Das Oleum wird im allgemeinen in einer solchen Menge eingesetzt, dass das darin enthaltende $SO_3$ in einem Überschuss von 3 bis 10 Mol, bevorzugt 4 bis 6 Mol, bezogen auf 1 Mol 4-Chlorbenzaldehyd, vorliegt.

Man verwendet dabei Oleum, das 35 bis 70 Gew.% freies $SO_3$ enthält, wobei die $SO_3$-Konzentration im Oleum bereits vorliegen kann oder aber erst durch Zugabe von $SO_3$ zu einer Lösung des Aldehyds in Schwefelsäure oder Oleum mit niedrigerem Gehalt an freiem $SO_3$ eingestellt werden kann.

Üblicherweise wird das erfindungsgemässe Verfahren so durchgeführt, dass man das Oleum vorlegt und dann den 4-Chlorbenzaldehyd in das Oleum einträgt.

Es ist aber auch möglich, den Benzaldehyd zunächst in Schwefelsäure oder in Oleum mit einem niedrigeren Gehalt an freiem $SO_3$ (z.B. 20%) zu lösen und diese Lösung in Oleum mit einem höheren Gehalt an $SO_3$ (z.B. 65%) einzutragen.

Eine weitere Möglichkeit der Durchführung besteht darin, 4-Chlorbenzaldehyd in Schwefelsäure gelöst vorzulegen und anschliessend das Oleum zuzugeben. Falls man 100 gew.-%iges Oleum, d.h. reines $SO_3$ verwendet, kann dieses sowohl in flüssiger Form als auch gasförmig, gegebenenfalls mit einem Inertgas verdünnt, zu der Reaktionslösung zugegeben werden.

Bei Vermischen der beiden Reaktionskomponenten steigt die Temperatur an. Man kann nun das Reaktionsgemisch während der Vermischung der beiden Reaktionskomponenten durch Kühlen auf Temperaturen von 0 bis 40°C, bevorzugt 10 bis 30°C, halten und nach der Vermischung die Temperatur auf 70 bis 110°C steigern oder aber die Vermischung der Reaktionspartner ohne Kühlung durchführen. Dabei steigt die Temperatur des Reaktionsgemisches von selbst auf Reaktionstemperatur. Die Reaktionstemperatur wird solange aufrechterhalten, bis sich der eingesetzte Benzaldehyd vollständig umgesetzt hat.

Die Reaktionszeit ist abhängig von der Stöchiometrie der eingesetzten Reaktionspartner und der Temperatur und beträgt im allgemeinen 1 bis 12 Stunden. Nachdem sich der Aldehyd vollständig umgesetzt hat, gibt man das Reaktionsgemisch auf Wasser oder Eis bzw. versetzt das Reaktionsgemisch mit Wasser oder Eis und arbeitet auf.

Die Isolierung der freien 2-Chlor-5-formylbenzolsulfonsäure kann durch Einstellen einer Schwefelsäurekonzentration von 40 bis 60 Gew.%, bevorzugt 45 bis 55 Gew.%, in der Hydrolysemischung bewerkstelligt werden. Die Einstellung der Schwefelsäurekonzentration kann so durchgeführt werden, dass dem Reaktionsgemisch die berechnete Menge Wasser oder Eis hinzugegeben wird. Nach Kühlen des Hydrolysegemisches auf –10°C bis 30°C, bevorzugt 0 bis 10°C, fällt die freie 2-Chlor-5-formylbenzolsulfonsäure aus und wird abgesaugt.

Man erhält die 2-Chlor-5-formylbenzolsulfonsäure als kristallines Trihydrat.

Es ist überraschend, dass die Isolierung der 2-Chlor-5-formylbenzolsulfonsäure als solche gelingt, da bekanntermassen die freien Sulfonsäuren von Benzaldehyden in Wasser und Schwefelsäure sehr leicht löslich sind. Ihre Isolierung gelingt nur in aufwendiger Weise durch Versetzen des Reaktionsgemisches mit Bariumcarbonat und Isolierung des Bariumsalzes, aus dem anschliessend mit stöchiometrischen Mengen Schwefelsäure die Benzaldehydsulfosäure freigesetzt wird (DE-PS 98 229, Ber. Dtsch. Chem. Ges. 16, 150 (1883)).

Unerwarteterweise enthält die nach dem erfindungsgemässen Verfahren hergestellte und als Trihydrat isolierte Chlorbenzaldehydsulfonsäure nur noch 3–5% Schwefelsäure. Daher ist es möglich, durch Neutralisation der isolierten Säure mit beliebigen anorganischen oder organischen Basen, wie Natriumhydroxid, Kaliumcarbonat, Natriumbicarbonat, Natriumsulfit, Ammoniak, Triethylamin und/oder Morpholin, alle gewünschten Salze in einfacher Weise und in hoher Reinheit herzustellen. Das so hergestellte Natrium-Salz hat z.B. einen Gehalt von 89,2%. Dieser hohe Gehalt ist bisher nur durch aufwendige Operationen, wie Aussalzen, Filtrieren und Waschen des Niederschlags, erneutes Auflösen, Nachneutralisieren und Eindampfen der Lösung zu erreichen (vgl. DE-OS 2 201 857).

Selbstverständlich kann die 2-Chlor-5-formylbenzolsulfonsäure auch direkt als Natriumsalz, Kaliumsalz oder Ammoniumsalz isoliert werden.

Man kann diese Salze der 2-Chlor-5-formylbenzolsulfonsäure isolieren, indem man zu der Hydrolyselösung z.B. Natriumchlorid, Kaliumchlorid, Natriumsulfat, Natriumsulfit, Ammoniumchlorid oder Ammoniumsulfat gibt. Das hierbei ausfallende Salz der 2-Chlor-5-formylbenzolsulfonsäure wird abgetrennt und in einer konzentrierten wässrigen Lösung des zur Fällung benutzten Salzes verrührt. Nach Neutralisation mit Basen wie z.B. Natronlauge, Kalilauge oder Ammoniak und erneutem Abtrennen, z.B. durch Filtrieren, erhält man ein Salz, das nach Trocknung direkt weiterverarbeitet werden kann.

Weiterhin ist es möglich, Salze der 2-Chlor-5-formylbenzolsulfonsäure durch partielle oder vollständige Neutralisation des Hydrolysegemisches zu isolieren. Bei Verwendung von z.B. Natriumhydroxid, Natriumbicarbonat und/oder Natriumcarbonat erhält man eine Suspension, aus der man das Natriumsalz der 2-Chlor-5-formylsulfonsäure in einfacher Weise beispielsweise durch Filtrieren abtrennen kann. Das so isolierte Salz kann nach Trocknung ohne weitere Reinigung weiterverarbeitet werden.

Die Verwendung von Calciumhydroxid, Calciumoxid oder Calciumcarbonat als Base ist natürlich auch möglich, jedoch ist diese Arbeitsweise aufwendiger als die Neutralisation mit z.B. Natriumhydroxid.

Die Vorteile des erfindungsgemässen Verfahrens liegen in den guten Ausbeuten, die im Vergleich zu der in der DE-OS 2 201 857 angegebenen Ausbeute um ca. 5 bis 10% besser sind, und den kurzen Reaktionszeiten, die sich im Vergleich zum Stand der Technik auf 1/2 bis 1/20 verkürzen. Ein weiterer Vorteil ist die einfache Isolierung der freien Sulfonsäure, wodurch sich die Herstellung ihrer Salze mit hohen Reinheitsgraden sehr vereinfacht.

Weiterhin überrascht es, dass bei den erfindungsgemässen hohen Reaktionstemperaturen nur geringe Oxidation des 4-Chlorbenzaldehyds zur Benzoesäure eintritt.

Es ist nämlich aus DE-PS 98 229 bekannt, dass bei hohen Reaktionstemperaturen Aldehyde bei der Sulfonierung leicht oxidiert werden.

Die nach dem erfindungsgemässen Verfahren hergestellte 2-Chlor-5-formylbenzolsulfonsäure und ihre Salze sind u.a. wertvolle Ausgangsprodukte für optische Aufheller auf der Basis von Bis-stilbenverbindungen (vgl. DE-OS 2 202 857).

Die nachfolgenden Beispiele sollen das erfindungsgemässe Verfahren erläutern, ohne es jedoch auf diese Beispiele einzuschränken.

Beispiel 1

Unter kräftigem Rühren wird 1 Mol bei 60°C geschmolzener 4-Chlorbenzaldehyd (99%ig) flüssig unter die Oberfläche von 554 g Oleum 65 (4,5 Mol SO$_3$) eingeleitet, wobei die Temperatur bei 10 bis 20°C gehalten wird. Die Mischung wird in 30 Minuten auf 85°C erhitzt und 3 Stunden bei 85°C gerührt. Dann wird auf 500 g Eis gegeben und auf –10°C gekühlt. Die ausgefallene Sulfonsäure wird abgesaugt. Man erhält 255,8 g 2-Chlor-5-formylbenzolsulfonsäure-trihydrat (Gehalt 91,7%). Ausbeute: 85,6% der Theorie. Fp: 98–100°C.

Beispiel 2

In 150 g Schwefelsäure werden bei 40°C 141 g (1 Mol) 4-Chlorbenzaldehyd gelöst und anschliessend in 40 Minuten 400 g (5 Mol) 100%iges SO$_3$ zugetropft. Es wird 5 Stunden bei 80°C gerührt und hydrolysiert. Man erhält 262 g 81,6%iges 2-Chlor-5-formylbenzolsulfonsäure-trihydrat. Ausbeute: 78% der Theorie.

**Beispiel 3**

In 985 g Oleum 65 (8 Mol SO₃) werden 141 g (1 Mol) 4-Chlorbenzaldehyd bei 10 bis 30°C eingetragen und 2 Stunden bei 90°C gerührt. Nach Hydrolyse und Absaugen bei 0°C erhält man 246 g 2-Chlor-4-formylbenzolsulfonsäure-trihydrat (Gehalt 89,0%). Ausbeute: 80% der Theorie.

**Beispiel 4**

255,8 g (0,856 Mol) 91,7%iges 2-Chlor-5-formyl-benzolsulfonsäure-trihydrat, isoliert nach Beispiel 1, wird in 150 ml H₂O gelöst und bei 60°C mit NaHCO₃ neutralisiert. Die Lösung wird zum Trocknen eingedampft und das Salz im Vakuum bei 150°C getrocknet. Es werden 232 g Natriumsalz der 2-Chlor-5-formylbenzolsulfonsäure (Gehalt 89,2%) erhalten. Ausbeute: 99,7% der Theorie.

**Beispiel 5**

Die Reaktion wird durchgeführt, wie in Beispiel 1 angegeben. Anschliessend wird auf 1060 g Wasser gegeben und mit 697 ml 45%iger NaOH neutral gestellt. Die Suspension wird auf 35°C gekühlt und nach 2 Stunden abgesaugt. Man erhält 256,5 g Natriumsalz der 2-Chlor-5-formylbenzolsulfonsäure (Gehalt 73,6%). Ausbeute: 78% der Theorie.

**Beispiel 6**

Die Sulfonierung wird durchgeführt, wie in Beispiel 1 angegeben. Anschliessend wird auf 1450 g Eis gegeben und danach 700 g Na₂SO₄ zugesetzt. Der Niederschlag wird bei 20°C abgesaugt und in 500 ml 12%iger Na₂SO₄-Lösung aufgeschlämmt. Man neutralisiert mit 20%iger Natronlauge, saugt ab und trocknet das Salz. Man erhält 254,4 g Natriumsalz der 2-Chlor-5-formylbenzolsulfonsäure (Gehalt 75,6%). Ausbeute: 79,5% der Theorie.

**Patentansprüche**

1. Verfahren zur Herstellung von 2-Chlor-5-formylbenzolsulfonsäure durch Umsetzung von 4-Chlorbenzaldehyd mit einem Überschuss an Oleum bei erhöhter Temperatur, dadurch gekennzeichnet, dass man die Umsetzung bei Temperaturen von 70 bis 110°C und bei einer Oleumkonzentration von 35 bis 70 Gew.% durchführt.

2. Verfahren nach Anspruch 1, dadurch gekennzeichnet, dass man die Umsetzung bei Temperaturen von 75 bis 90°C durchführt.

3. Verfahren nach Anspruch 1 und 2, dadurch gekennzeichnet, dass man die Umsetzung bei Temperaturen von 80 bis 85°C durchführt.

4. Verfahren nach den Ansprüchen 1 bis 3, dadurch gekennzeichnet, dass man die freie 2-Chlor-5-formylbenzolsulfonsäure durch Einstellen einer Schwefelsäurekonzentration von 40 bis 60 Gew.% im Hydrolysegemisch isoliert.

5. Verfahren nach Ansprüchen 1 bis 4, dadurch gekennzeichnet, dass man die freie 2-Chlor-5-formylbenzolsulfonsäure nach Abkühlung des Hydrolysegemisches auf −10 bis 30°C als Trihydrat isoliert.

6. Verfahren nach den Ansprüchen 1 bis 3, dadurch gekennzeichnet, dass man das Natriumsalz der 2-Chlor-5-formylbenzolsulfonsäure durch partielle oder vollständige Neutralisation des Hydrolysegemisches mit Natriumhydroxid, Natriumbicarbonat und/oder Natriumcarbonat isoliert.

7. Verfahren nach den Ansprüchen 1 bis 3, dadurch gekennzeichnet, dass man die Salze der 2-Chlor-5-formylbenzolsulfonsäure durch Aussalzen aus der sauren Hydrolysemischung isoliert.

8. Verfahren nach den Ansprüchen 1 bis 3 und 7, dadurch gekennzeichnet, dass man als Salze Natriumchlorid, Natriumsulfat, Natriumsulfit, Natriumbisulfit, Kaliumchlorid oder Kaliumsulfat verwendet.

**Claims**

1. Process for the preparation of 2-chloro-5-formylbenzenesulphonic acid by reacting 4-chlorobenzaldehyde with an excess of oleum at an elevated temperature, characterised in that the reaction is carried out at temperatures from 70 to 110°C and with an oleum concentration of 35 to 70% by weight.

2. Process according to Claim 1, characterised in that the reaction is carried out at temperatures from 75 to 90°C.

3. Process according to Claim 1 and 2, characterised in that the reaction is carried out at temperatures from 80 to 85°C.

4. Process according to Claims 1 to 3, characterised in that the free 2-chloro-5-formylbenzenesulphonic acid is isolated by adjusting the sulphuric acid concentration in the hydrolysis mixture to 40 to 60% by weight.

5. Process according to Claims 1 to 4, characterised in that the free 2-chloro-5-formylbenzenesulphonic acid is isolated as the trihydrate, after the hydrolysis mixture has been cooled to −10 to 30°C.

6. Process according to Claims 1 to 3, characterised in that the sodium salt of 2-chloro-5-formyl-benzenesulphonic acid is isolated by partial or complete neutralization of the hydrolysis mixture with sodium hydroxide, sodium bicarbonate and/or sodium carbonate.

7. Process according to Claims 1 to 3, characterised in that the salts of 2-chloro-5-formylbenzenesulphonic acid are isolated by salting out from the acidic hydrolysis mixture.

8. Process according to Claims 1 to 3 and 7, characterised in that sodium chloride, sodium sulphate, sodium sulphite, sodium bisulphite, potassium chloride or potassium sulphate are used as salts.

**Revendications**

1. Procédé pour la fabrication d'acide 2-chloro-5-formylbenzènesulfonique par réaction du 4-chlorobenzaldéhyde avec un excès d'oléum à température élevée, caractérisé en ce que l'on effectue la réaction à des températures de 70 à 110°C et à une concentration de l'oléum de 35 à 70% en poids.

2. Procédé selon la revendication 1, caractérisé en ce que l'on effectue la réaction à des températures de 75 à 90°C.

3. Procédé selon la revendication 1 ou 2, caractérisé en ce que l'on effectue la réaction à des températures de 80 à 85°C.

4. Procédé selon les revendications 1 à 3, caractérisé en ce que l'acide 2-chloro-5-formylbenzènesulfonique libre est isolé par réglage de la concentration en acide sulfurique dans le mélange d'hydrolyse à 40–60% en poids.

5. Procédé selon les revendications 1 à 4, caractérisé en ce que l'on isole l'acide 2-chloro-5-formylbenzènesulfonique à l'état de trihydrate après refroidissement du mélange d'hydrolyse à −10 à 30°C.

6. Procédé selon les revendications 1 à 3, caractérisé en ce que l'on isole le sel de sodium de l'acide 2-chloro-5-formylbenzènesulfonique par neutralisation partielle ou totale du mélange d'hydrolyse par l'hydroxyde de sodium, le bicarbonate de sodium et/ou le carbonate de sodium.

7. Procédé selon les revendications 1 à 3, caractérisé en ce que l'on isole les sels de l'acide 2-chloro-5-formylbenzènesulfonique par relargage du mélange d'hydrolyse acide.

8. Procédé selon les revendications 1 à 3 et 7 en ce que l'on utilise comme sels le chlorure de sodium, le sulfate de sodium, le sulfite de sodium, le bisulfite de sodium, le chlorure de potassium ou le sulfate de potassium.